# FASCICULE DE BREVET EUROPEEN

(11) **EP 2 521 522 B1**
(45) Date de publication et mention de la délivrance du brevet: **28.05.2014**
(21) Numéro de dépôt: 10810770.7
(22) Date de dépôt: 29.12.2010
(51) Int. Cl.: A61B 5/00, G01N 33/50, A61F 13/00, A61F 13/84, A61L 15/56, G01N 33/52, A61F 13/02

(54) **PROCÉDÉ DE DÉTECTION D'UN ANALYTE DANS UN FLUIDE CORPOREL**
VERFAHREN FÜR DEN NACHWEIS VON ANALYTEN IN EINER KÖRPERFLÜSSIGKEIT
METHOD FOR DETECTING ANALYTE IN A BODILY FLUID

(30) Priorité: 04.01.2010 FR 1000007
(43) Date de publication de la demande: 14.11.2012
(73) Titulaire: Commissariat à l'Énergie Atomique et aux Énergies Alternatives, 75015 Paris (FR)
(72) Inventeur: MARCHAND, Gilles, F-38119 Pierre-Châtel (FR); MARCOUX, Pierre, F-38120 Saint Egrève (FR)
(74) Mandataire: Talbot, Alexandre
(86) Numéro de dépôt international: PCT/FR2010/000882
(87) Numéro de publication internationale: WO 2011/080427

(56) Documents cités:
- EP-A1- 0 430 608
- EP-A1- 1 790 982
- WO-A2-02/30478
- CA-A1- 2 497 724
- US-A- 4 548 906
- US-A- 5 899 856
- US-A1- 2002 091 347
- US-A1- 2007 142 762

## Description

### Domaine technique de l'invention.

L'invention concerne un procédé de détection d'un analyte dans un liquide corporel.

### État de la technique

De nombreux travaux ont été réalisés pour détecter de façon qualitative la présence d'un analyte dans un fluide corporel.

Depuis quelques années, on utilise des techniques de colorimétrie dans le domaine médical pour répondre, en particulier, à un besoin de détermination de l'évolution d'une plaie et pour décrire les germes qui se développent dans l'exsudat de la plaie.

À l'origine de la classification des bactéries, le test de Gram est une méthode répandue qui permet d'identifier et de différencier les bactéries grâce à un test de coloration réalisé en solution. Cette méthode nécessite de prélever du liquide corporel puis de réaliser un test Gram in vitro. Ce test repose sur les caractéristiques des membranes et de la paroi des bactéries.

D'autres indicateurs colorimétriques sont utilisés pour détecter non pas la bactérie en elle-même mais un ou plusieurs produits générés au cours de la croissance de la bactérie. Ainsi, dans l'article « Spot Indole Test : Évaluation of Four Reagents » (Journal of Clinical Microbiology, 1982, 589-592), J.M. Miller et al. décrit un papier imprégné par une solution contenant le réactif de Kovacs. Le réactif de Kovacs est connu pour détecter des bactéries dites « indoles positives » dans une solution. Ces bactéries génèrent de l'indole au cours de leur croissance, sous-produit de l'hydrolyse du tryptophane par la tryptophanase. Le diméthyl-amino-4-benzaldéhyde contenu dans le réactif de Kovacs réagit avec l'indole et forme un composé coloré en rouge.

Récemment, des pansements se basant sur différents types d'indicateurs ont été proposés. Les pansements utilisés pour des plaies doivent être changés à intervalles réguliers, par exemple, afin de maintenir une certaine humidité favorisant la cicatrisation de la plaie et de garder propres les parties entourant la plaie et la plaie ou pour changer le type de pansement appliqué en fonction du stade de cicatrisation de la plaie.

Au cours de la cicatrisation, le suivi de l'évolution d'une plaie est une partie importante du travail quotidien du personnel médical car il permet de connaître, notamment, l'état de cicatrisation de la plaie et fournit une indication sur le moment où le pansement doit être remplacé. Les plaies concernées sont, par exemple, les esquarres, les plaies diabétiques, les plaies formées par un ulcère, une brûlure, une chirurgie ou une greffe, les tumeurs de la peau ou les maladies génétiques. La gestion courante des plaies est, généralement, réalisée approximativement de façon visuelle et odorante. Dans le cas d'une charge bactérienne importante au sein de la plaie, un cortège de signes inflammatoires propres à l'infection et à la réaction inflammatoire de défense de l'organisme font leur apparition : exsudat abondant, épais, voire nauséabond et coloré en fonction du germe, érythème péri-lésionnel avec douleur spontanée et réaction oedémateuse.

Le document US-A-2002091347 décrit un pansement qui indique quand il doit être changé en fonction du taux d'humidité du pansement. Ce pansement comprend, successivement, un revêtement externe, une couche imperméable à l'eau, un indicateur coloré et une garniture de pansement. La garniture de pansement se présente sous la forme d'une couche destinée à être en contact avec la plaie et est imperméable à l'eau jusqu'à ce qu'elle se sature en eau et devienne alors perméable à l'eau. L'eau traverse alors le pansement et atteint l'indicateur coloré qui change de :couleur au contact de l'eau.

D'autres pansements ont été proposés visant à détecter une élévation de température caractéristique d'une infection. Ces travaux sont basés sur le principe que l'application d'un pansement sur une zone de la peau ou d'une plaie entraîne une augmentation de la température dans cette zone. L'accumulation de métabolites dans un espace confiné, combinée à une augmentation de la pression autour de ladite zone due à la présence du pansement, provoque un afflux de sang responsable de l'élévation en température. Le document US-A-5181905 divulgue, par exemple, un pansement comprenant un indicateur de température constitué par une bande de détection de la température, à cristaux liquides.

Cette approche a le désavantage d'être peu discriminante quant aux causes provoquant cette élévation de température, notamment quant à la nature des métabolites présents dans ladite zone et, par conséquent, sur le besoin ou non de changer le pansement.

Le document US-B-7364918 propose un capteur colorimétrique à base d'assemblages de polydiacétylène et d'un récepteur ainsi qu'un procédé d'utilisation de ce capteur pour détecter un analyte. Le récepteur est choisi pour interagir spécifiquement avec l'analyte. Ce récepteur est incorporé dans un réseau polymère ène-yne conjugué avant ou après polymérisation. La structure du polymère conjugué ainsi formé a la capacité de changer de conformation en fonction des interactions du récepteur avec l'analyte. Les perturbations induites sur la structure du squelette de polymère conjugué entraînent alors un changement de couleur. Le capteur colorimétrique peut indiquer, par exemple, la présence de bactéries Gram-négatives et Gram-positives dans des liquides biologiques. Les auteurs proposent également de combiner ce capteur colorimétrique à un pansement, pour détecter la présence d'une infection, par exemple, en l'intégrant dans un pansement sous forme d'une couche en contact direct ou indirect avec la plaie, mais selon ce document, le capteur est toujours au contact de l'exsudat. Néanmoins, un capteur basé sur un simple changement conformationnel d'un polymère peut entraîner un nombre important de faux-positifs.

D'autres travaux ont été réalisés pour détecter, plus largement, la présence d'un analyte à partir d'un fluide corporel collecté sur un individu comme un fluide vaginal, du sang ou de l'urine. On peut citer, par exemple, les documents US-A-5910447, US-A-4548906 et US-A-2007258860 qui proposent des détecteurs et des procédés de détection d'ammoniaque ou d'amines sous forme gazeuse.

On peut également citer, le document US-A-5899856 qui décrit un procédé de détection de l'éthanol ou d'un métabolite de l'éthanol à partir de la sueur transpirée par un individu. Le procédé comporte, en particulier, la collecte d'éthanol grâce à un patch dermique positionné sur la peau, pour absorber l'éthanol transpiré, puis l'extraction par désorption de l'éthanol ou du métabolite contenu dans le patch dermique suivie de l'analyse du liquide extrait.

On peut également citer le document WO 02/30478 A2 qui décrit un pansement pour la mise en oeuvre d'un procédé de détection d'un analyte dans un liquide corporel (l'exsudat d'une plaie), basé sur la détection, par un détecteur chromogène, d'un métabolite issu d'une activité enzymatique caractéristique dudit microbe et caractérisé par le fait que ledit détecteur chromogène ne soit pas en contact direct avec le liquide corporel.

Néanmoins, les solutions proposées ne sont pas applicables directement sur l'individu, pour une analyse en temps réel.

### Objet de l'invention

L'invention a pour but de proposer un procédé de détection de la présence d'un analyte présent dans un fluide corporel donnant des informations qualitatives fiables et précises, et pouvant être mis en oeuvre en dehors du cadre d'un laboratoire.

Un pansement pour la mise en oeuvre de ce procédé, peu coûteux, donnant une indication fiable, précise et rapide sur la présence d'un analyte dans un liquide corporel est décrit.

Selon l'invention, ce but est atteint par un procédé de détection selon les revendications annexées.

En particulier, ce but est atteint par le fait qu'un détecteur est placé au-dessus d'une zone de la peau d'un individu ou d'un animal contenant le liquide corporel, par le fait qu'on détecte ensuite au moins un métabolite issu du métabolisme ou de la dégradation de l'analyte, uniquement sous forme gazeuse, par mise en contact du détecteur avec le métabolite gazeux, ledit métabolite gazeux étant issu de l'évaporation d'au moins une partie dudit métabolite et ladite mise en contact produisant un signal détectable optiquement et par le fait que la détection est réalisée sans contact entre le liquide corporel et le détecteur.

### Description sommaire des dessins

D'autres avantages et caractéristiques ressortiront plus clairement de la description qui va suivre des modes particuliers de réalisation de l'invention donnés à titre d'exemples non limitatifs et représentés aux dessins annexés, dans lesquels :
- Les figures 1 à 5 représentent, schématiquement et en coupe, différents modes particuliers de réalisation d'un pansement selon l'invention.
- La figure 6 représente, schématiquement et en coupe, un dispositif de test de détection de l'indole sur un pansement selon l'invention.
- La figure 7 représente, schématiquement, un dispositif de test selon l'axe AA de la figure 6.

### Description des modes de réalisation particuliers de l'invention

Selon un mode de réalisation particulier, un procédé de détection d'un analyte dans un liquide corporel comporte le positionnement d'un détecteur au-dessus d'une zone de la peau d'un individu ou d'un animal contenant le liquide corporel puis la détection d'au moins un métabolite, uniquement sous forme gazeuse, issu du métabolisme ou de la dégradation de l'analyte. La détection est réalisée par mise en contact du détecteur placé au-dessus de la zone de la peau contenant le liquide corporel, avec le métabolite gazeux. Le métabolite gazeux détecté est issu de l'évaporation d'au moins une partie du métabolite. La mise en contact du détecteur avec le ou les métabolite(s) gazeux produit un signal détectable optiquement. La détection est réalisée sans contact entre le liquide corporel et le détecteur. En effet, les liquides corporels sont susceptibles de contenir de nombreux analytes qui pourraient interagir avec le détecteur si ce dernier était en contact du fluide corporel. Cela peut générer des faux-positifs. On a donc intérêt à éviter tout contact entre le fluide corporel et le détecteur. De plus, l'absence de contact évite toute contamination du liquide corporel par une substance contenue dans le détecteur.

Le liquide corporel peut être, par exemple, de la sueur, du sang, les larmes, les sécrétions vaginales, la lymphe, la salive, l'urine, ou l'exsudat d'une plaie de la peau d'un individu ou d'un animal. En particulier, le liquide corporel est constitué par de l'exsudat provenant d'une plaie de la peau d'un individu ou d'un animal.

Le terme "analyte" inclut, sans s'y limiter, les organismes pathogènes et non pathogènes, les substrats enzymatiques, les anticorps, les antigènes, les protéines, les peptides, les bactéries, les virus, protozoaires, enzymes, acides aminés, lipides, sucres, hormones, les parasites ou les champignons ou les composants du liquide corporel. On peut également ajouter les petites molécules telles que l'urée, le terme petites molécules désignant des molécules dont le poids moléculaire est inférieur à 500 g/mol.

L'analyte est, avantageusement, une bactérie. On citera, à titre indicatif et de façon non limitative, les bactéries gram-positives du genre Staphylococcus comme le staphylocoque Staphylococcus aureus ou les bactéries gram-négatives du genre Pseudomonas comme la Pseudomonas aeruginosa et les bactéries dites « indoles positives » du genre Escherichia comme l'Escherichia coli.

Certaines familles de bactéries sont bien connues pour pouvoir générer des métabolites au cours de leur croissance. Un certain nombre de ces métabolites sont volatils c'est-à-dire ont une tendance à s'évaporer, par exemple, au contact de l'air. Ainsi, dans l'article "Détection of volatile métabolites produced by bacterial growth in blood culture media by selected ion flow tube mass spectrometry (SIFT-MS)", (Microbiol. Methods, 2006, 65, 361-365), Allardyce r.a. et al., a mis en évidence la production de métabolites lors du métabolisme des staphylocoques dorés, notamment des métabolites comme de l'éthanol, acétone, acétaldéhyde, ammoniaque, acide acétique, diméthyle sulfure, méthane thiol, dihydrogène sulfuré, indole, aminoacétophénone, triméthylamine et hexanal. De même, il a été montré que le Pseudomonas aeruginosa émet du diméthyle sulfure, du méthane thiol et du dihydrogène sulfuré.

Par ailleurs, les bactéries dites « indoles positives » comme Escherichia coli, Aeromonas hydrophilia et certaines Citrobacters sont également connues pour générer, au cours de leur croissance, de l'indole qui est également un produit volatil.

L'émission de ces métabolites volatils produits lors du métabolisme de la bactérie peut constituer une véritable empreinte ou signature de la bactérie. On entend par métabolite volatil tout métabolite susceptible de passer à l'état gazeux.

D'autres analytes présents dans un liquide corporel peuvent, également, se dégrader, par exemple par oxydation ou sous l'action de bactéries ou d'enzymes, et libérer alors un ou plusieurs métabolites volatils. À titre d'exemple, on peut citer l'urée qui est présente dans la sueur et qui peut se décomposer en ammoniac et en gaz carbonique.

Le métabolite volatil issu du métabolisme ou de la dégradation de l'analyte est, avantageusement, un composé organique volatil ou "COV" (« Volatile Organic Compound » en anglais).

Ainsi, en détectant le ou les métabolites gazeux issus du métabolisme ou de la dégradation de l'analyte, il est possible de détecter la présence de l'analyte si ce métabolite ou la combinaison des métabolites est spécifique à l'analyte. En utilisant un détecteur spécifique, on peut détecter également les métabolites d'une même famille de composés, par exemple, l'éthanol et le méthanol ou la putrescine et la cadavérine ou H₂S et CH₃SH.

Selon un mode de réalisation particulier, pour favoriser la détection, une première étape du procédé de détection consiste à former un espace confiné par application d'un élément de confinement comportant le détecteur sur une zone de la peau d'un individu ou d'un animal contenant du liquide corporel. Le détecteur est, de préférence, placé au-dessus de la zone de la peau d'un individu ou d'un animal contenant le liquide corporel.

On entend par espace confiné, un espace délimité par l'élément de confinement et la zone de la peau de l'individu ou de l'animal. En revanche, ce terme n'implique pas nécessairement qu'il y ait un espace vide entre l'élément de confinement et le liquide corporel. L'élément de confinement peut, éventuellement, être en contact avec le liquide corporel : ainsi, le liquide corporel n'entre pas en contact avec le détecteur.

L'élément de confinement peut être au moins en partie perméable à certaines espèces gazeuses.

Au moins une partie du métabolite issu du métabolisme ou de la dégradation de l'analyte s'évapore progressivement après application de l'élément de confinement sur la peau. La partie du métabolite non-évaporée reste dans le liquide corporel sous forme dissoute. L'accroissement de la concentration du métabolite sous forme gazeuse par rapport à la concentration du même métabolite sous forme liquide dépend de plusieurs paramètres, notamment, de la nature du métabolite, des dimensions de l'élément de confinement et de la quantité de liquide corporel. Cette concentration dépend de la loi de Henry exprimant un ratio entre la concentration d'une espèce en phase liquide sur la concentration de cette même espèce en phase gazeuse.

Le métabolite sous forme gazeuse pénètre dans l'espace confiné jusqu'à entrer en contact avec le détecteur.

La mise en contact entre le détecteur et le métabolite gazeux produit un signal détectable optiquement. On entend par détectable optiquement tout signe perceptible qui peut être observé ou lu, directement ou indirectement, par un moyen optique. Lors de la mise en contact, le détecteur et le métabolite gazeux interagissent. Cette interaction est responsable d'une modification des propriétés optiques du détecteur.

Le détecteur est, avantageusement, spécifique à un ou plusieurs métabolites à analyser c'est-à-dire qu'il produit un ou plusieurs signaux détectables optiquement au contact du ou des métabolites à analyser présents sous forme gazeuse. Le détecteur produit, avantageusement, un signal proportionnel à la quantité de métabolite mis en contact pour une analyse qualitative et quantitative.

Le détecteur est, de préférence, choisi parmi un détecteur chromogène ou un détecteur fluorogène. L'interaction induite par la mise en contact du métabolite gazeux avec le détecteur provoque une modification du spectre d'absorption ou d'émission. Une réaction de type covalente entre le métabolite gazeux et le détecteur est privilégiée car elle donne lieu à la création d'au moins une liaison covalente stable dans le temps et évite toute perte d'informations au cours du temps. De même, l'interaction de type covalente entre le métabolite gazeux et le détecteur peut provoquer une modification du spectre plus importante que dans le cas de la création de liaisons faibles, par exemple, de type van der Waals améliorant ainsi la sensibilité du détecteur.

Dans le cas d'un détecteur chromogène, la mise en contact du métabolite gazeux avec le détecteur produit un changement de couleur d'au moins une partie du détecteur. Le détecteur chromogène présente l'avantage d'une lecture directe, visible à l'oeil nu, de la présence ou non de l'analyte dans le fluide corporel.

Dans le cas d'un détecteur fluorogène, la mise en contact du métabolite gazeux avec le détecteur produit une émission fluorescente d'au moins une partie du détecteur. Dans ce cas, un moyen d'excitation de la fluorescence, notamment une source de lumière adaptée comme une lampe à ultraviolets, peut être nécessaire pour détecter le rayonnement de fluorescence.

Selon un mode de réalisation particulier, le détecteur comporte une sonde sensible au métabolite et un support. On entend par sonde toute molécule ou substance ayant une affinité de liaison pour une cible. La sonde est responsable, en particulier, de la propriété chromogène ou fluorogène du détecteur comme décrit ci-dessus.

La sonde possède une ou plusieurs fonctions qui interagissent avec le métabolite constituant la cible de sorte qu'une fois liée au métabolite, la sonde produit un signal détectable optiquement. La sonde réagit, de préférence, avec au moins un métabolite de manière irréversible et sélective, de préférence par liaison covalente. La réaction de la sonde et du métabolite forme, avantageusement, un composé absorbant ou fluorescent. Le détecteur fluorogène présente généralement l'avantage d'une plus grande sensibilité.

La sonde peut être spécifique à un métabolite volatil ou à une classe de métabolites possédant une fonction ou une réactivité particulière. La faculté de la sonde à interagir avec une cible sera dépendante du métabolite constituant la cible. En fonction du métabolite à détecter, l'homme de l'art est en mesure de déterminer la sonde connue à utiliser et est capable de mettre en oeuvre les étapes de synthèse et/ou d'insertion dans le support et/ou de greffage sur le support selon tout procédé connu pour obtenir le détecteur adéquat.

À titre de premier exemple, la sonde peut être le 4-Aminopent-3-en-2-one qui est un composé connu pour détecter les aldéhydes légers comme le formaldéhyde, sous forme de composés organiques volatils (VOCs) présents dans l'air. À titre d'exemple, le 4-Aminopent-3-en-2-one réagit avec le formaldéhyde selon la réaction (1) suivante :

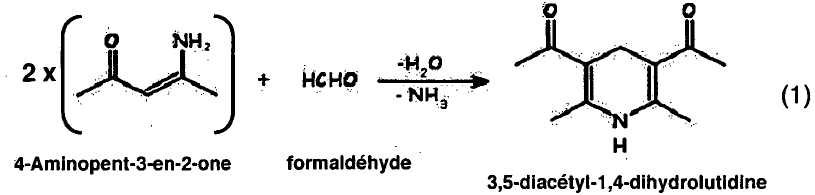

Le 3,5-diacétyl-1,4-dihydrolutidine obtenu est fluorescent.

Par ailleurs, le 4-(diméthylaminobenzaldéhyde) (noté "DMABA") connu comme réactif de James et le 4-diméthylaminocinnamaldéhyde (noté "DMACA") connu comme réactif de Kovacs permettent de détecter l'indole en solution, respectivement, selon les réactions (2) et (3) suivantes : 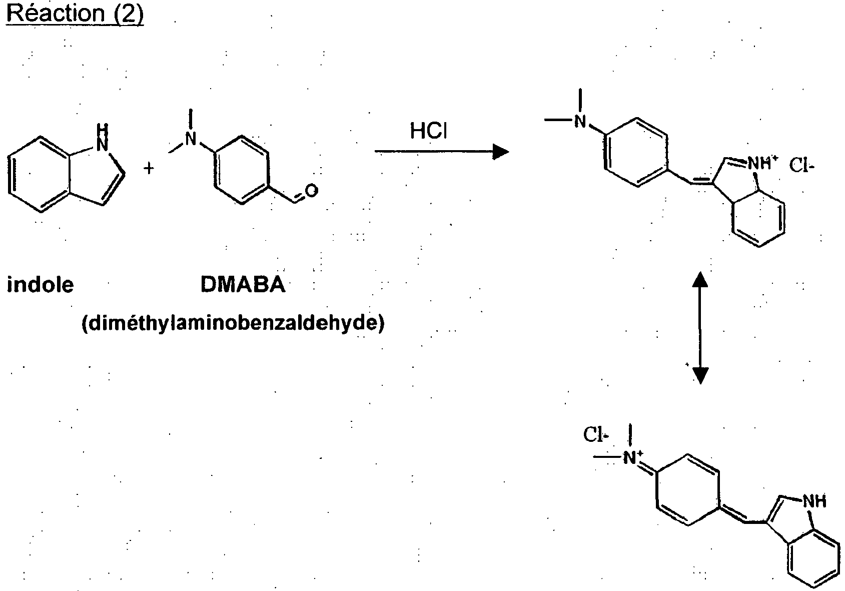 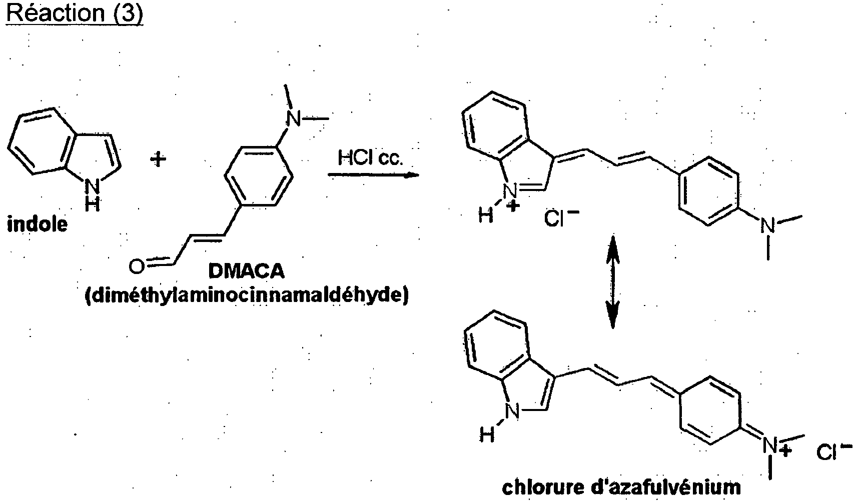 L'interaction entre la cible indole et une sonde de type DMABA ou DMACA conduit à des chlorures d'azafulvénium, qui sont des produits absorbants.

À titre de second exemple, la cible peut être la 2'-aminoacétophénone connue comme étant un composé organique volatil spécifique de la Pseudomonas aeruginosa. La détection du 2'-aminoacétophénone peut être réalisée par la réaction de Friedländer. En effet, la 2'-aminoacétophénone réagit avec la cyclohexanone en présence d'acide acétique en quantité catalytique, pour donner un dérivé de la quinoléine selon la réaction (4) suivante :

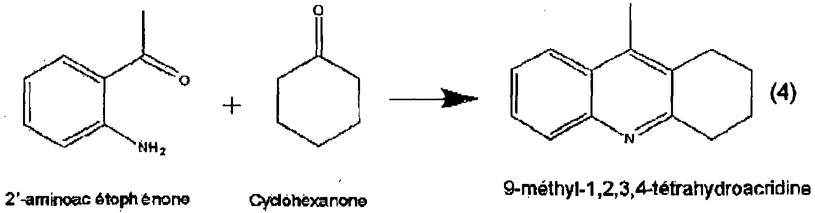

Les dérivés de la quinoléine, en particulier, la 9-méthyl-1,2,3,4-tétrahydroacridine est une molécule colorée.

Cette détection peut, également, être réalisée par condensation de la 2'-aminoacétophénone sur un aldéhyde tel que le benzaldéhyde en présence d'acide acétique en quantité catalytique Le produit réactionnel obtenu est alors une imine stabilisée, 1-(2-(benzylidèneamino)phényl)éthanone c'est-à-dire conjuguée et colorée selon la réaction (5) suivante :

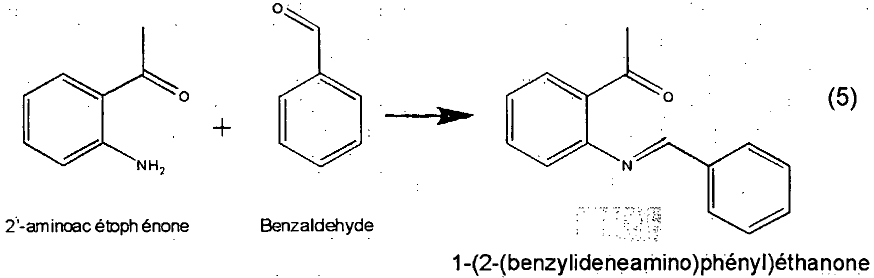

La sonde peut être incorporée dans le support selon tout procédé connu, par exemple, par simple piégeage ou adsorption dans la structure du support. La sonde peut également être liée au support par liaison covalente ou par adsorption mettant en oeuvre des liaisons faibles, telles qu'une liaison de type Van der Waals, ionique, hydrogène, électrostatique ou dative.

Selon une variante, la sonde peut être constituée par le support lui-même, notamment lorsque le métabolite volatil est coloré. Son adsorption sur le support entraîne alors une modification du spectre d'absorption du support.

Le support est, avantageusement, transparent ou translucide pour faciliter la lecture du résultat de la détection.

Le support est, de préférence, perméable au métabolite sous forme gazeuse. Le support est, avantageusement, un support poreux pour permettre une meilleure diffusion du métabolite au sein du support et favoriser le contact du métabolite avec la sonde. La porosité du support permet également de contrôler la diffusion des espèces gazeuses au sein du détecteur notamment en assurant le tri en fonction de la taille des différents métabolites gazeux. La porosité du support est un paramètre qui peut jouer un rôle sur la sélectivité de la détection.

Le support est, avantageusement, un polymère. On entend par polymère, les polymères solubles et insolubles ainsi que les co-polymères. Le support peut être choisi parmi les polymères organiques. Par exemple, le support peut être une résine macroporeuse comme une résine polystyrène macroporeuse. De préférence, le polymère est choisi parmi les polymères connus non toxiques et/ou biocompatibles.

Il peut s'agir également d'un oxyde poreux, de préférence non métallique, par exemple à base d'oxyde de silicium.

Selon un mode particulier de réalisation, le support est un polymère poreux pour permettre la circulation du métabolite sous forme gazeuse au travers du squelette polymère. La surface spécifique du polymère poreux est, de préférence, supérieure ou égale à 50m²/g, de préférence supérieure ou égale à 500 m²/g, pour augmenter la probabilité d'interaction entre la sonde et le métabolite sous forme gazeuse et améliorer ainsi la sensibilité de la détection.

La sonde peut être liée au polymère formant le support par liaison covalente, selon tout procédé connu, par exemple par post-fonctionnalisation d'un polymère ou co-polymérisation de monomères préalablement fonctionnalisés.

Selon un autre mode de réalisation particulier, le support est un polymère ayant une forte capacité à solubiliser les composés gazeux. Ainsi, outre le rôle de support, le polymère peut également servir à absorber et/ou stocker les métabolites gazeux. Ainsi, au moins une partie du métabolite, sous forme gazeuse, mis en contact avec le détecteur peut se solubiliser dans le polymère puis réagir avec la sonde sous forme solubilisée.

Le polymère est, de préférence, choisi parmi tout polymère gel connu ayant une bonne capacité à dissoudre des gaz comme les polymères fortement hydratés.

Le polymère est, par exemple, choisi parmi un hydrogel tel que d'agarose, un hydrogel, un alginate, un gel d'acide hyaluronique, le polydiméthylsiloxane, la cellulose, le polyéthylène glycol, le polyalcool de vinyle (noté "PVA"), le poly(propylène fumarate), les poly(alpha-hydroxyesters), les poly(orthoesters), les polyanhydrides, les poly(phosphazènes), le poly(propylène fumarate), les poly(ester amides), les poly(éthylène fumarate), l'acide polylactique, l'acide polyglycolique, le polycaprolacton (noté "PCL"), et le polydioxanone (noté "PDO), le poyuréthane et la carboxylméthylcellulose.

Le polymère gel est, avantageusement choisi parmi un alginate, un hydrogel, un polymère gel à base d'huile de silicone et un organogel gel fluoré ou siliconé, tel qu'un gel de Cholesteryl anthraquinone-2-carboxylate et de polyméthylsiloxane, ou un gel de 1,3:2,4-dibenzylidene-Sorbitol et d'octamethylcyclotetrasiloxane ou un gel de diamide aromatique à chaîne perfluorée et de perfluorotributylamine, un tel gel étant décrit dans la publication de J. Loiseau et al. (Tetrahedron, 2002, 4049-4052).

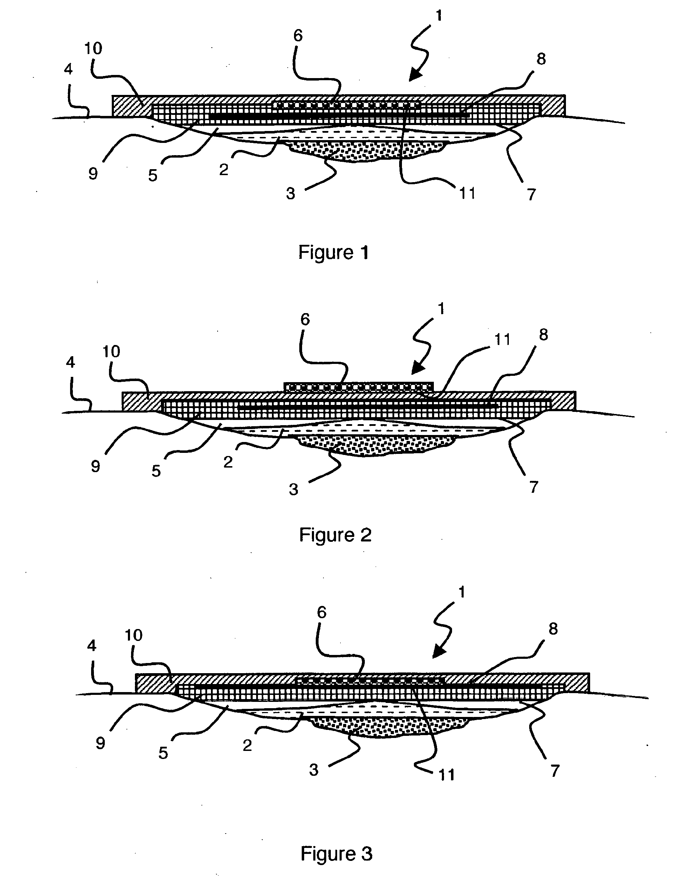

Selon un mode de réalisation préférentiel représenté aux figures 1 à 5, l'élément de confinement comporte un pansement 1. En particulier, l'élément de confinement peut être un pansement 1. Le liquide corporel 2 est, de préférence, constitué par de l'exsudat provenant d'une plaie 3 de la peau 4. Le pansement 1 est appliqué sur une zone de la peau 4 d'un individu ou d'un animal contenant le liquide corporel 2 pour former un espace confiné 5. L'espace confiné 5 est délimité par le pansement 1 (en haut à la figure 1) et la peau 4 de l'individu ou de l'animal recouverte par le pansement 1 (en bas à la figure 1). Le pansement 1 est solidaire de la peau 4.

Comme représenté aux figures 1 à 5, le pansement 1 permettant la mise en oeuvre du procédé de détection décrit ci-dessus comporte un détecteur 6, une face de recouvrement 7 de la peau 4 d'un individu ou d'un animal et des moyens de protection 8 compris entre la face de recouvrement 7 et le détecteur 6. Le détecteur 6 est tel que décrit précédemment, en particulier chromogène ou fluorogène.

L'espace confiné 5 est, plus précisément, délimité par la face de recouvrement 7 du pansement 1 et la surface de la peau 4 recouverte par la face de recouvrement 7. Le détecteur 6 est situé dans ou sur le pansement 1 et agencé, de préférence, au-dessus du liquide corporel 2 de façon à favoriser la mise en contact du métabolite sous forme gazeuse qui se volatilise et le détecteur 6.

Les moyens de protection 8 sont inertes vis-à-vis du métabolite, perméables au métabolite sous forme gazeuse et imperméables au liquide corporel 2. La présence de moyens de protection 8 permet d'éviter tout contact entre le liquide corporel 2 et le détecteur 6. Les moyens de protection 8 constituent une barrière physique empêchant le liquide corporel 2 d'atteindre le détecteur 6. Les moyens de protection 8 sont, avantageusement, formés par au moins une couche d'un ou plusieurs matériaux perméables au métabolite sous forme gazeuse et imperméables au liquide corporel 2. Avantageusement, les moyens de protection 8 comportent une couche de polymère hydrophobe, par exemple, un polyuréthane, ce polymère étant naturellement hydrophobe.

Selon un premier mode de réalisation particulier représenté à la figure 1, le pansement 1 comporte un pansement primaire 9, un pansement secondaire 10 et le détecteur 6 situé à l'interface du pansement primaire et le pansement secondaire, respectivement 9 et 10. Comme représenté à la figure 1, le pansement 1 peut être formé par un empilement de couches adjacentes comportant successivement le pansement secondaire 10, le détecteur 6 et le pansement primaire 9 (de haut en bas à la figure 1).

Le pansement primaire 9 est généralement au contact ou potentiellement au contact de l'exsudat. Il peut être constitué d'un matériau choisi parmi : un textile tissé ou non tissé comme le tulle, la gaze de viscose, coton, tricot de polyamide, trame de polyester, un polymère ou polymère gel tel que le polyuréthane, le carboxyméthylcellulose, l'alginate, collagène, de gaze, ou de l'acétate, de la viscose, charbon actif enveloppé dans une feuille non tissé textile, par exemple une pièce de gaze. Le pansement primaire 9 peut être une pièce de gaze tissée et/ou non-tissée en coton, éventuellement, hydrophile. Une pièce de gaze tissée et non-tissée en coton constitue une pièce de gaze ouatée.

Le pansement primaire 9 peut assurer une ou plusieurs des fonctions suivantes : pompage de liquide corporel 2, fonction antibactérienne, hydratation, cicatrisation, anti-odeur.

Le pansement secondaire 10 assure, essentiellement, une fonction de maintien. Le pansement secondaire 10 peut comprendre toute bande adhésive connue ou un bandage conventionnel. Le pansement secondaire 10 est, avantageusement, transparent ou translucide pour permettre une lecture directe du signal émis par le détecteur 6. Ainsi, la lecture de la présence ou l'absence d'un ou de plusieurs analytes dans le liquide corporel 2 peut être réalisée sans enlever le pansement 1 de la peau 4.

Généralement, un pansement est constitué d'un tel pansement primaire 9 et d'un tel pansement secondaire 10, les matériaux et fonctions du pansement primaire 9 et du pansement secondaire 10 ayant été précédemment décrits.

Dans ce premier mode de réalisation, le détecteur fait partie du pansement primaire 9, des moyens de protection, faisant aussi partie du pansement primaire 9 empêchant la migration du fluide corporel 2 vers le détecteur 6. On verra, dans les modes de réalisation suivants, que le détecteur 6 peut également faire partie du pansement secondaire 10, les moyens de protection étant alors disposés dans le pansement primaire 9 et/ou dans le pansement secondaire 10.

On verra également que, lorsque le détecteur 6 fait partie d'un pansement primaire 9, le pansement primaire 9 peut être constitué d'un même matériau, le détecteur 6 étant placé dans une première partie hydrophobe de ce matériau, les moyens de protection étant réalisés avec une seconde partie hydrophile de ce même matériau.

Selon une variante non représentée, le pansement secondaire 10 possède une fenêtre transparente en vis-à-vis et au-dessus du détecteur 6 (en haut à la figure 1), pour pouvoir visualiser à l'oeil nu l'éventuel changement de couleur du détecteur 6 lorsque le pansement 1 est appliqué sur la peau 4.

Le pansement 1 comporte, avantageusement, le détecteur 6 ayant une face de détection 11 située en vis-à-vis du fluide corporel 2, la face de recouvrement 7 en vis-à-vis du liquide corporel 2 et des moyens de protection 8 pour isoler le détecteur 6 du liquide corporel 2.

Comme représenté à la figure 1, les moyens de protection 8 sont disposés entre la face de détection 11 et la face de recouvrement 7 et, de préférence, placés dessous et en vis-à-vis le détecteur 6 de façon à constituer une barrière physique à la migration du liquide corporel 2 vers le détecteur 6. Avantageusement, les moyens de protection 8 sont intégrés dans le pansement primaire 9 (figure 1).

Comme représenté à la figure 1, la face de recouvrement 7 peut être en contact avec le liquide corporel 2, notamment lorsque le liquide corporel 2 détecté est de l'exsudat provenant d'une plaie 3. Dans ce cas, la face de recouvrement 7 couvre, de préférence, la zone de la peau 4 où se trouve la plaie 3.

Le détecteur 6, de préférence un détecteur 6 chromogène ou fluorogène, produit un signal détectable optiquement au contact d'au moins un métabolite, sous forme gazeuse, issu du métabolisme ou de la dégradation de l'analyte contenu dans le liquide corporel 2 comme décrit ci-dessus.

Comme représenté à la figure 1, le détecteur 6 peut être constitué par une couche déposée sur le pansement primaire 9 selon tout procédé connu, par exemple, comme décrit précédemment par imprégnation d'un polymère gel contenant une sonde sur le pansement primaire 9, de préférence textile. À titre d'exemple, le détecteur 6 peut être réalisé par mélange d'au moins la sonde et le polymère gel suivi du dépôt du mélange ainsi obtenu sur le pansement primaire 9 textile du pansement 1. Le mélange sonde/polymère s'imprègne dans le pansement primaire 9 puis se solidifie pour former le détecteur 6.

Le pansement 1 peut, également, comporter plusieurs détecteurs 6, identiques ou différents, spécifiques à un ou plusieurs métabolites pour une détection multi-paramétrique, une meilleure reproductibilité et une meilleure sensibilité des résultats.

Selon un second mode de réalisation particulier représenté à la figure 2, le pansement 1 est identique au premier mode de réalisation particulier (figure 1) à l'exception du fait que le détecteur 6 est disposé sur une face du pansement secondaire 10 qui constitue alors la face de détection 11. Le pansement secondaire 10 doit, dans ce cas, être au moins en partie perméable au métabolite sous forme gazeuse.

Selon un troisième mode de réalisation particulier représenté à la figure 3, le pansement 1 est identique à celui du premier mode de réalisation (figure 1) à l'exception du fait que le pansement 1 est constitué par un empilement de couches adjacentes comportant successivement le pansement secondaire 10, le détecteur 6, les moyens de protection 8 et le pansement secondaire 10 (de haut en bas à la figure 3). Le détecteur 6 est disposé sur la couche constituant les moyens de protection 8. La face de détection 11 est alors au contact d'au moins une partie des moyens de protection 8.

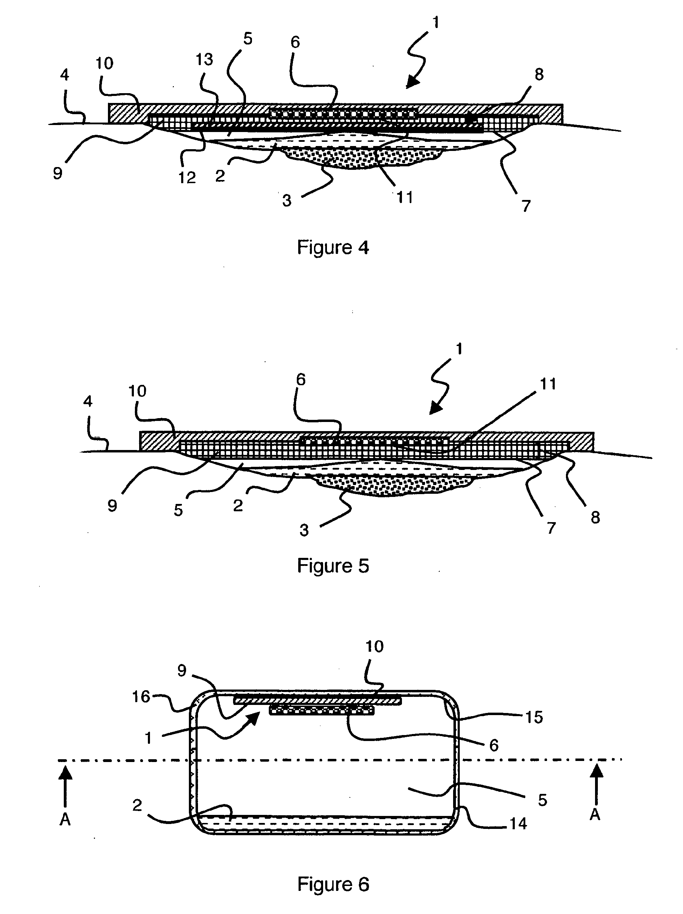

Selon un quatrième mode de réalisation représenté à la figure 4, le pansement 1 est identique à celui du premier mode de réalisation (figure 1) à l'exception du fait que la face de recouvrement 7 est, avantageusement, constituée au moins en partie par les moyens de protection 8. À titre d'exemple, les moyens de protection 8 sont formés par une couche externe 12 d'un polymère hydrophile, par exemple un polyuréthane rendu hydrophile par tout moyen connu, et une couche interne 13 d'un polymère hydrophobe tel qu'un polyuréthane, ce matériau étant naturellement hydrophobe. La couche externe 12 est destinée à être en contact avec le liquide corporel 2 sur la peau 4 de l'individu ou l'animal et la couche interne 13 est disposée en vis-à-vis du détecteur 6. Le caractère hydrophile de la couche externe 12 favorise l'interaction avec le fluide corporel 2 et son imprégnation à l'intérieur du pansement 1 et permet, avantageusement, de rapprocher le liquide corporel 2 du détecteur 6 dans le pansement 1. Le caractère hydrophobe de la couche interne 13 rend les moyens de protection 8 imperméables au liquide corporel 2 et empêche la migration du liquide corporel 2 vers le détecteur 6. Les moyens de protection 8 sont, par exemple, constitués par une couche en polyuréthane, pour constituer la couche interne 13. La couche 13 en polyuréthane protège le détecteur 6 en formant une barrière physique à l'entrée du liquide corporel 2 dans le pansement primaire 9 au-delà de la couche de protection tout en autorisant la diffusion des espèces gazeuses.

Selon un cinquième mode de réalisation représenté à la figure 5, le mode de réalisation diffère des autres modes de réalisation en ce que les moyens de protection 8 sont, avantageusement, formés par une épaisseur de garniture du pansement primaire 9. La garniture joue alors le rôle de barrière physique à la progression du liquide corporel 2 au sein du pansement 1 et isole le détecteur 6 du liquide corporel 2. Pour une pièce de gaze tissée, la garniture est formée classiquement par une succession de couches de gaze tissée. L'épaisseur de la garniture du pansement primaire 9 doit être suffisamment importante pour constituer une entrave à l'imprégnation du liquide corporel 2 au sein du pansement 1 jusqu'au détecteur 6. La garniture peut être rendue localement hydrophobe au voisinage du détecteur 6. Le pansement primaire 9 a une épaisseur, avantageusement, comprise entre 1 mm et 50 mm, de préférence entre 1 mm et 10 mm.

Selon un autre exemple, le pansement primaire est constitué de polyuréthane, les moyens de protection étant constitués de polyuréthane rendu hydrophile, l'autre partie, contenant le détecteur, étant constitué de polyuréthane, ce matériau étant naturellement hydrophobe.

### EXEMPLES

### Exemple 1 : réalisation d'un détecteur 6 formé d'un polymère poreux et d'une sonde piégée dans la structure du polymère

300mg de 4-(diméthylamino)benzaldéhyde (DMABA) sont dissous dans 10ml de méthanol. 2mL d'acide chlorhydrique 1M sont ajoutés à la solution sous agitation.

1 g d'agarose est dissout dans 20 ml d'eau à 80°C sous agitation. Après dissolution, la solution contenant le DMABA est additionnée à la solution d'agarose à 80°C. Après retour à température ambiante (environ 25°C), on obtient le détecteur 6 sous forme d'un gel blanchâtre.

### Exemple 2 : réalisation d'un pansement 1 contenant un détecteur 6

Un pansement 1 commercialisé sous la marque URGO Discret est utilisé pour la détection de l'indole. Ce pansement 1 comporte un pansement primaire 9 contenant une pièce de gaze textile et un pansement secondaire 10 adhésif, transparent.

Une goutte de 10µl du détecteur 6 synthétisé selon l'exemple 1 est préalablement chauffée à 80°C puis déposée sur le pansement primaire 9 textile du pansement 1. En revenant à température ambiante, le détecteur 6 absorbé par la partie textile du pansement primaire 9 se solidifie sous forme d'une couche de gel blanchâtre.

### Test de détection de l'indole

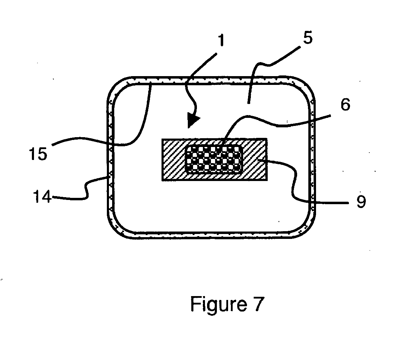

Comme représenté aux figures 6 et 7, une solution d'indole dans l'eau est préparée à 10⁻¹ M. 1 ml est placé dans récipient 14 transparent de type plaque à puits. Le pansement 1 obtenu à l'exemple 1 est collé sur la paroi interne 15 du couvercle 16 de la plaque à puits 14 par sa partie adhésive de sorte que la couche formée par imprégnation du détecteur 6 à la surface du pansement primaire 9 est en face de la solution d'indole. Le détecteur 6 n'est pas en contact avec cette solution d'indole. Après 10 minutes à température ambiante, le détecteur 6 devient rose montrant ainsi la réaction entre l'indole volatilisé sous forme gazeuse et le détecteur 6.

### Exemple 3 : réalisation d'un pansement 1 contenant deux détecteurs 6

300mg de 4-(diméthylamino)cinnamaldéhyde sont dissous dans 10 ml de méthanol. 2ml d'acide chlorhydrique 1M sont ajoutés à la solution sous agitation. 1 g d'agarose est dissout dans 20 ml d'eau à 80°C sous agitation. Après dissolution, la solution contenant le DMACA est additionnée à la solution d'agarose à 80°C puis, avant retour à température ambiante, une goutte de 30µl est déposée sur le pansement primaire 9 textile d'un pansement 1 de la marque URGO Discret, commercialisé par URGO. La goutte est alors absorbée par la partie textile du pansement primaire 9. Après retour à température ambiante, on obtient le détecteur 6 sous forme d'une couche de gel orangé-marron.

Le même mode opératoire est réalisé avec du DMABA à l'exception du fait que le détecteur 6 est déposé sur le même pansement primaire 9 du pansement URGO Discret mais à un endroit différent. Après retour à température ambiante, on obtient un détecteur 6 sous forme d'une couche de gel blanchâtre (détecteur 6 DMABA/agarose) adjacente à une couche de gel orangé-marron (détecteur 6 DMACA/agarose).

### Cultures des bactéries

Une colonie d'une souche *Escherichia* coli ATCC 11775- Indole positive commercialisée par la société LGC Standards, notée EC5, est mise en pré-culture dans 4mL de milieu LB à 30°C sous agitation pendant environ 15 h. Une colonie sur boîte d'agar LB est suspendue dans 4mL de LB (x2 tubes), agitée puis maintenue à une température de 30°C en statique pendant 15 h.

Le même mode opératoire est réalisé avec deux autres souches: *Hafnia* alvei ATCC 13337- Indole négative commercialisées par la société LGC standards, notée HA4, et *Pseudomonas putida* ATCC 12633- Indole négative commercialisées par la société LGC standards, notée PP6.

### Préparation du milieu Tryptophane agar:

La préparation du milieu liquide Tryptophane/agar est réalisée par mélange de 3,2 g/200 ml de DEV Tryptophan Broth (FLUKA 31406) et 3 g/200 ml d'agar (FLUKA 05039) suivi de la mise en autoclave à 121°C et 2,2 bars pendant 20 min.

Le milieu est sorti de l'autoclave encore liquide c'est-à-dire à une température comprise entre environ 60°C et 70°C, pour être directement coulé en boîte de Pétri (FALCON 353001, Becton Dickinson, diamètre 35 x hauteur 10 mm).

### Culture en liquide

Tubes de pré-culture le plus concentré, DO à 550 nm:
pour HA4: DO(1/5x) = 0,16, soit une DO effective de 0,8;
pour EC5: DO(1/5x) = 0,25, soit une DO effective de 1,25;
pour PP6: DO(1/5x) = 0,29, soit une DO effective de 1,5.

On inocule sur une base de 1/50^{ème} pour une DO de 0,7 soit un inoculum au 1/70^{ème} (60 µL de pré-culture pour 4 mL de LB). La température au début de la culture est de 37°C, sous agitation à 250 rpm.

Après 2 heures de culture, on obtient :
HA4: DO(1x) = 0,34 soit 1,35.10⁸ cfu/mL
EC5: DO(1x) = 0,59 soit 1,40.10⁸ cfu/mL
PP6: DO(1x) = 0,15 soit environ 5.10⁷ cfu/mL

Pour inoculer une boîte de DEV Tryptophan/agar, on étale 20 µL de culture avec une öse soit 1 à 3 millions de cfu par boîte.

### Test de détection de l'indole

Trois boîtes 14 ont été préparées avec les souches EC5, HA4 et PP6. Un pansement 1, selon l'exemple 3, est collé sur la paroi interne 15 du couvercle 16 des boîtes de Pétri 14 contenant respectivement EC5, HA4 et PP6 de sorte que les détecteurs 6, DMABA/agarose et DMACA/agarose, ne touchent pas le milieu 2 puis on incube chaque boîte de Pétri 14 à une température de 37°C.

Après 3h00 de culture à 37°C, on obtient un changement de couleur des deux détecteurs 6. Le détecteur 6 contenant le DMACA passe du orangé-marron au vert et le détecteur 6 contenant le DMABA passe du blanchâtre au rose uniquement dans la boîte 14 contenant EC5 (indole positive). On constate l'efficacité du procédé de détection pour la détection de l'indole issu du métabolisme des bactéries de type indole positive.

### Exemple 4: réalisation d'un détecteur 6 formé d'un polymère poreux contenant du cyclohexanone ou du benzaldéhyde

Une solution contenant 2.3ml de tétraéthoxysilane de formule Si(OC₂H₅)₄) (TEOS), 0.6mL d'eau, 3.6mL d'éthanol, 0.1mL d'acide acétique et 1.2 mL de cyclohexanone ou de benzaldéhyde constituant la molécule sonde est préparée dans un pilulier.

### Exemple 5 : réalisation d'un pansement 1 contenant le détecteur 6 selon l'exemple 4

Après homogénéisation, la solution de l'exemple 4 est déposée sur le pansement primaire 9 d'un pansement 1 identique à l'exemple 2. Après absorption de la solution sur la pièce textile, le pansement 1 est chauffé à 70°C pendant 3 heures. Le détecteur 6 contenant la molécule sonde est obtenu après séchage à l'air ambiant, sous forme d'une monocouche solide et poreuse.

### Détection du 2'-aminoacétophénone

Une solution de 2'-aminoacétophénone dans de l'eau est préparée à 0.5M. 5 mL sont placés dans un flacon muni d'un couvercle. Le pansement 1 obtenu à l'exemple 5 est collé sur la paroi interne du couvercle de façon à orienter le pansement primaire 9 contenant le détecteur 6, face à la solution de 2'-aminoacétophénone, sans toutefois mettre en contact le détecteur 6 avec la solution de 2'-aminoacétophénone. Après 72 minutes à 37°C; le détecteur 6 devient jaune si la molécule sonde est de la cyclohexanone ou orangé si la molécule sonde est du benzaldéhyde. Trois tests témoins ont été réalisés à titre comparatif selon un mode opératoire identique. Les résultats obtenus sont répertoriés dans le tableau ci-déssous :

| **N°** | **Solution** | **Molécule sonde** | **Coloration du détecteur** |
|---|---|---|---|
| **1** | 2'-aminoacétophénone 0.5M | - | - |
| **2** | 2'-aminoacétophénone 0.5M | cyclohexanone | jaune |
| **3** | Eau | cyclohexanone | - |
| **4** | 2'-aminoacétophénone 0.5M | benzaldéhyde | orangée |
| **5** | Eau | benzaldéhyde | - |

On constate que les tests témoins restent incolores alors que les flacons 2 et 4 détectent effectivement la présence de 2'-aminoacétophénone.

La présente invention n'est pas limitée aux exemples et modes de réalisation particuliers purement illustratifs. Des variations sont possibles dans le cadre de la protection revendiquée, en particulier, le pansement 1 peut comporter plusieurs couches notamment des couches hydrophiles, en polymère ou textiles, assurant notamment une tenue mécanique et/ou améliorant les conditions de détection du ou des métabolites. Par ailleurs, lorsque le liquide corporel est de la sueur, l'élément de confinement peut être différent d'un pansement.

La finalité du procédé de détection de la présente invention est d'indiquer la présence ou non d'un ou plusieurs analytes dans un liquide corporel. Le procédé de détection présente l'avantage d'une détection sensible et discriminante par rapport aux différents analytes à analyser. Le procédé permet, en particulier, par constatation ou non d'un signal, une analyse qualitative et avantageusement quantitative de l'analyte, avec une sensibilité élevée et un risque de faux-positifs faible.

Le procédé de détection est simple à mettre en oeuvre, rapide et évite toute contamination du liquide corporel. En effet, le pansement permettant la mise en oeuvre du procédé contient un détecteur qui n'est pas en contact avec le liquide corporel et ne modifie pas, par conséquent, la composition du liquide corporel. En particulier, le pansement appliqué sur une plaie ne perturbe pas la cicatrisation et évite tout risque de toxicité ou de modification de la coloration du liquide corporel. Par ailleurs, le signal détecté par le procédé de détection peut fournir un résultat intermédiaire pertinent indiquant la nécessité de changer le pansement.

## Revendications

1. Procédé de détection d'un analyte dans un liquide corporel (2) **caractérisé en ce qu'**un détecteur (6) est placé au-dessus d'une zone de la peau (4) d'un individu ou d'un animal contenant le liquide corporel (2), **en ce qu'**on détecte ensuite au moins un métabolite issu du métabolisme ou de la dégradation de l'analyte, uniquement sous forme gazeuse, par mise en contact du détecteur (6) avec le métabolite gazeux, ledit métabolite gazeux étant issu de l'évaporation d'au moins une partie dudit métabolite et ladite mise en contact produisant un signal détectable optiquement et **en ce que** la détection est réalisée sans contact entre le liquide corporel (2) et le détecteur (6).

2. Procédé de détection selon la revendication 1, **caractérisé en ce que** le détecteur (6) est choisi parmi un détecteur chromogène et un détecteur fluorogène.

3. Procédé de détection selon l'une des revendications 1 et 2, **caractérisé en ce qu'**un espace confiné (5) est formé par application d'un élément de confinement comportant le détecteur (6), sur une zone de la peau (4) d'un individu ou d'un animal contenant du liquide corporel (2).

4. Procédé de détection selon la revendication 3, **caractérisé en ce que** l'élément de confinement comporte un pansement (1).

5. Procédé de détection selon l'une quelconque des revendications 1 à 4, **caractérisé en ce que** le liquide corporel (2) est constitué par de l'exsudat provenant d'une plaie (3) de la peau (4) d'un individu ou d'un animal.

6. Procédé de détection selon l'une quelconque des revendications 1 à 5, **caractérisé en ce que** le détecteur (6) comporte une sonde sensible au métabolite et un support.

7. Procédé de détection selon la revendication 6, **caractérisé en ce que** la sonde est incorporée dans le support.

8. Procédé de détection selon la revendication 6, **caractérisé en ce que** la sonde est liée au support par liaison covalente.

9. Procédé de détection selon l'une quelconque des revendications 6 à 8, **caractérisé en ce que** le support est perméable au métabolite sous forme gazeuse.

10. Procédé de détection selon l'une quelconque des revendications 6 à 9, **caractérisé en ce que** le support est transparent ou translucide.

11. Procédé de détection selon l'une quelconque des revendications 6 à 10, **caractérisé en ce que** le support est un polymère.

12. Procédé de détection selon la revendication 11, **caractérisé en ce que** le polymère est poreux et a une surface spécifique supérieure ou égale à 50 m²/g.

13. Procédé de détection selon la revendication 11, **caractérisé en ce que** le polymère est choisi parmi un alginate, un hydrogel, un polymère gel à base d'huile de silicone et un polymère gel fluoré.

## Patentansprüche

1. Verfahren zum Nachweis eines Analyten in einer Körperflüssigkeit (2), **dadurch gekennzeichnet, dass** ein Detektor (6) oberhalb eines Bereiches der Haut (4) einer Person oder eines Tiers, die bzw. das die Körperflüssigkeit (2) enthält, platziert wird, dass anschließend wenigstens ein Metabolit, welcher aus dem Stoffwechsel oder aus dem Abbau des Analyten hervorgegangen ist, einzig in gasförmiger Form durch Inkontaktbringen des Detektors (6) mit dem gasförmigen Metaboliten nachgewiesen wird, wobei der gasförmige Metabolit aus der Verdampfung wenigstens eines Teils des Metaboliten hervorgegangen ist und das Inkontaktbringen ein optisch nachweisbares Signal erzeugt, und dass der Nachweis ohne Kontakt zwischen der Körperflüssigkeit (2) und dem Detektor (6) vollzogen wird.

2. Nachweisverfahren nach Anspruch 1, **dadurch gekennzeichnet, dass** der Detektor (6) aus einem chromogenen Detektor und einem fluorogenen Detektor ausgewählt ist.

3. Nachweisverfahren nach einem der Ansprüche 1 und 2, **dadurch gekennzeichnet, dass** ein eingeschlossener Raum (5) durch Aufbringen eines den Detektor (6) umfassenden Einschließungselements auf einen Bereich der Haut (4) einer Person oder eines Tiers, die bzw. das Körperflüssigkeit (2) enthält, gebildet wird.

4. Nachweisverfahren nach Anspruch 3, **dadurch gekennzeichnet, dass** das Einschließungselement einen Verband (1) umfasst.

5. Nachweisverfahren nach einem der Ansprüche 1 bis 4, **dadurch gekennzeichnet, dass** die Körperflüssigkeit (2) durch aus einer Wunde (3) der Haut (4) einer Person oder eines Tiers stammendes Exsudat gebildet ist.

6. Nachweisverfahren nach einem der Ansprüche 1 bis 5, **dadurch gekennzeichnet, dass** der Detektor (6) eine gegenüber dem Metaboliten empfindliche Sonde und einen Träger umfasst.

7. Nachweisverfahren nach Anspruch 6, **dadurch gekennzeichnet, dass** die Sonde in den Träger integriert ist.

8. Nachweisverfahren nach Anspruch 6, **dadurch gekennzeichnet, dass** die Sonde durch kovalente Bindung mit dem Träger verbunden ist.

9. Nachweisverfahren nach einem der Ansprüche 6 bis 8, **dadurch gekennzeichnet, dass** der Träger für den Metaboliten in gasförmiger Form durchlässig ist.

10. Nachweisverfahren nach einem der Ansprüche 6 bis 9, **dadurch gekennzeichnet, dass** der Träger transparent oder durchscheinend ist.

11. Nachweisverfahren nach einem der Ansprüche 6 bis 10, **dadurch gekennzeichnet, dass** der Träger ein Polymer ist.

12. Nachweisverfahren nach Anspruch 11, **dadurch gekennzeichnet, dass** das Polymer porös ist und eine spezifische Oberfläche von mehr als oder gleich 50 m²/g aufweist.

13. Nachweisverfahren nach Anspruch 11, **dadurch gekennzeichnet, dass** das Polymer aus einem Alginat, einem Hydrogel, einem Gelpolymer auf der Basis von Silikonöl und einem fluorierten Gelpolymer ausgewählt ist.

## Claims

1. Method for detecting an analyte in a bodily fluid (2), **characterized in that** a detector (6) is placed above a zone of an individual's or animal's skin (4) containing the bodily fluid (2), **in that** at least one metabolite resulting from the metabolism or degradation of the analyte is then detected, only in gas form, by placing the detector (6) in contact with the gas-form metabolite, said gas-form metabolite resulting from the evaporation of at least a part of said metabolite and said contact producing an optically detectable signal and **in that** the detection is carried out without contact between the bodily fluid (2) and the detector (6).

2. Method according to claim 1, **characterized in that** the detector (6) is selected between a chromogene detector and a fluorogene detector.

3. Method according to any one of the claims 1 and 2, **characterized in that** a confined space (5) is formed by applying an element of confinement comprising the detector (6), on a zone of an individual's or animal's skin (4) containing the bodily fluid (2).

4. Method according to claim 3, **characterized in that** the element of confinement comprises a dressing (1).

5. Method according to any one of the claims 1 to 4, **characterized in that** the bodily fluid (2) consists of exudate from a wound (3) of an individual's or animal's skin (4).

6. Method according to any one of the claims 1 to 5, **characterized in that** the detector (6) comprises a probe sensitive to the metabolite and a support.

7. Method according to claim 6, **characterized in that** the probe is incorporated into the support.

8. Method according to claim 6, **characterized in that** the probe is bound to the support by a covalent bond.

9. Method according to any one of the claims 6 to 8, **characterized in that** the support is permeable for the gas-form metabolite.

10. Method according to any one of the claims 6 to 9, **characterized in that** the support is transparent or translucent.

11. Method according to any one of the claims 6 to 10, **characterized in that** the support is a polymer.

12. Method according to claim 11, **characterized in that** the polymer is porous and has a specific surface area higher than or equal to 50 m²/g.

13. Method according to claim 11, **characterized in that** the polymer is selected among an alginate, a hydrogel, a gel polymer containing silicone oil and a fluorinated gel polymer.
